# EUROPEAN PATENT APPLICATION

(11) **EP 4 484 927 A1**
(43) Date of publication of application: **01.01.2025**
(21) Application number: 23182911.0
(22) Date of filing: 30.06.2023
(51) Int. Cl.: G01N 21/31, G01N 21/33, G01N 21/3577, G01N 21/53, G01N 33/18

(54) **OPTICAL DEVICE FOR MEASURING TURBIDITY AND COLOUR CONTENT OF FRESH WATER OR SEA WATER**

(30) Priority: 29.06.2023 PT 2023118780
(71) Applicant: Universidade do Minho, 4704-553 Braga (PT)
(72) Inventor: VALENTE GONÇALVES, LUÍS MIGUEL, 4800-058 GUIMARÃES (PT); GUIMARÃES LOPES, PEDRO DAVID, 4800-058 GUIMARÃES (PT); RAMOS AIRES MOREIRA PENSO, CAMILA MARIA, 4800-058 GUIMARÃES (PT); SOUSA CARNEIRO, CÁTIA FILIPA, 4800-058 GUIMARÃES (PT)
(74) Representative: Patentree

(57) **Abstract**

It is disclosed an optical device for measuring turbidity and colour content of fresh water or sea water comprising a first structure comprising on its top surface a plurality of light emitters; a second structure comprising a plurality of light receivers; wherein said plurality of light emitters and plurality of light receivers comprise: at least one infra-red emitter and one infra-red receiver; at least one ultraviolet emitter and one ultraviolet receiver; at least one visible emitter and one visible receiver; wherein each light emitter emits an illumination beam with an angle from 10 to 80°; wherein the first structure and the second structure are at a pre-determined distance.

## Description

### TECHNICAL FIELD

The present disclosure relates to a device for measuring turbidity and colour content of water. In particular it related to an optical sensor for measuring underwater turbidity and colour content of fresh water or sea water.

### BACKGROUND

Protecting water resources is essential for mitigating the impacts of global warming. Preserving hydrological basins and implementing sustainable practices in water use play a critical role in maintaining natural ecosystems. In-situ sensors are a highly valuable tool for continuously monitoring these resources. They provide essential information that enables responsible authorities to act promptly and minimize the impact of any issues.

Rivers provide water resources that sustain agricultural and industrial activities and play a vital role in surrounding ecosystems. However, the exploitation of these water bodies by economic sectors has contributed to a decline in river water quality. [1] Aquifer pollution, partly caused by illegal discharges of industrial effluents, untreated domestic sewage, construction and mineralization waste, and livestock activity residues, poses significant risks to ecosystems and public health. [2] This problem is partly driven by the high cost of waste treatment, which is often lower than the fines paid by industries. It is imperative to raise public awareness regarding the preservation and conservation of rivers, with the aim of preventing illegal discharges and encouraging reports. Presently, the majority of quality analyses are conducted through the collection of samples for laboratory analysis, gathered at various intervals throughout the year, to obtain a more comprehensive view and detect sources of contamination. However, in-situ analysis confers several advantages in this field, allowing for real-time monitoring, which enables prompt and effective problem resolution. Additionally, in-situ sensors are more cost-effective, as they eliminate the entire process of collection, transportation, and laboratory analysis. The creation of alert parameters, namely water color changes, that allow for the identification of deviations from standard values for further testing and analysis is crucial. When used in conjunction with other techniques, they enable rapid alerting of authorities. Meaning they are excellent allies in minimizing environmental impact and protecting aquatic life. In this way, colorimeters, when paired with efficient communication systems, represent a highly useful tool for combatting illegal discharges.

In Europe, the European Environment Agency (EEA) publishes regular reports on the state of Europe's waters, including rivers. According to their 2020 report, some of the key findings related to river pollution include [3]:
Nutrient pollution from agro activities, specifically nitrogen and phosphorus;
Organic pollution, which can come from sources such as industrial discharges and sewage treatment plants;
Heavy metals, including mercury, cadmium, and lead that can enter the water from industrial activities and historic pollution;
Emerging pollutants, such as pharmaceuticals and microplastics.

Many of these pollutants have the ability to induce alterations in the spectral colour of water, enabling the detection of these parameters through water colour analysis. Several sensors have been developed in this field. For instance, in [4], [5], the authors have devised in-situ sensors based on spectroscopy to measure nitrates and nitrites in marine environments. Similarly, in [6], an in-situ colorimetric analysis sensor has been developed to assess phosphate and nitrite levels in agricultural water. Additionally, there exist more versatile sensors, such as the one outlined in [7], where the authors have utilized mobile phone spectroscopy to detect orthophosphate, ammonia nitrogen, and heavy metals within the spectral range of 400 to 700 nm.

The technological advancements of recent years have allowed for the development of various spectroscopic methodologies to detect pollutants in aqueous samples. Absorption spectroscopy, measures light absorption at a specific wavelength, which allows for the identification of illegal discharges, as many pollutants have strong characteristic colors. These sensors are easy to use, low-cost, and require little maintenance, making them excellent for implementation in remote areas.

In modern times, various technologies based on spectral analysis have emerged for the determination of water quality parameters. Among them, hyperspectral image analysis has been utilized through satellite imagery to examine chlorophyll, suspended matter, and suspended colored organic matter. [8] However, this method requires the use of complex algorithms to perform accurate readings and corrections. Despite it can cover large areas, it's not adequate to acquire information about small rivers or streams, where vegetation covers the surface of the water. Furthermore, studies have also been conducted regarding the analysis of chemical oxygen demand, a crucial parameter in assessing water quality, using a cost-effective, portable spectral analysis sensor.[9] Additionally, another study employs a colorimeter to analyze nitrite concentrations, thereby investigating the health parameters of fish in aquaculture. In [10], it is designed a low-cost, portable colorimeter utilizing three LEDs and a light-dependent resistor. Notably, in [11], the authors utilized LEGO components to construct a swift and economical system based on LEDs for analyzing the concentration of Cu²⁺ in cuvettes.

Document WO02068940A1 discloses a turbidity sensor for measuring a full range of particulate content in a turbid environment, including a method thereof. The turbidity sensor includes a laser light source for emitting laser light through the particulate content. The turbidity sensor additionally includes at least one light-sensitive detector located proximate to the laser light source for the detection of light scattered from particles of the particulate content that come into contact with laser light emitted from the laser light source, thereby permitting the measurement of the turbidity of the turbid environment. A plurality of light-sensitive detectors may be employed, including a back scatter detector, a side scatter detector, a forward scatter detector, and a direct transmission detector. The light-sensitive detectors may be arranged in a geometric configuration of light-sensitive detectors with respect to the laser light source.

Document US4263511 discloses an in situ turbidity meter, for measuring water quality of a body of water comprising a pulsed infrared laser which illuminates the water, thereby producing scattered radiation from particles suspended in the water. A portion of the scattered radiation is collected and correlated to the turbidity of the water.

Document WO2013160877 discloses a portable multiparameter turbidity sensor based on fiber optics. The sensor quantifies optical transmission and dispersion in a fluid by nephelometry, using light emission at two or more wavelengths. It allows the concentration of suspended solids to be estimated, the type of sediment to be distinguished on the basis of colour, grain size classes to be differentiated, and partial concentrations of suspended solids to be identified and determined. The sensor comprises an emitter of one or more light wavelengths, a receiver for measuring transmission, a receiver for measuring dispersion, and a measurement cell with an inner cavity for receiving the fluid being analysed, these elements being arranged at predefined distances and predefined angles in relation to each other.

These facts are disclosed in order to illustrate the technical problem addressed by the present disclosure.

### GENERAL DESCRIPTION

The present disclosure relates to a sensor that aims to analyze water quality by detecting possible contaminations, considering variations in color and turbidity.

An optical transmission setup with eleven different excitation wavelengths ranging from 365-940 nm and a corresponding detection system is disclosed.

In an embodiment, the system uses a GSM antenna to transmit real-time information and alarms to a big data server.

It is disclosed an optical device for measuring turbidity and colour content of fresh water or sea water comprising
a first structure comprising on its top surface a plurality of light emitters;
a second structure comprising a plurality of light receivers;
wherein said plurality of light emitter and plurality of light receivers comprise:
   at least one infra-red emitter and one infra-red receiver;
   at least one ultraviolet emitter and one ultraviolet receiver;
   at least one visible emitter and one visible receiver;
wherein each light emitter emits an illumination beam with an angle from 10° to 80°;
wherein the first structure and the second structure are at a pre-determined distance;
wherein the first structure and the second structure form a chamber for receiving the fluvial or sea water.

In an embodiment, each light receiver of the plurality of light receivers receives at least part of the illumination beam of the correspondent light emitters.

In an embodiment, each light receiver of the plurality of light receivers receives the illumination beam of the correspondent plurality of light emitters.

In an embodiment, the number of infra-red emitter is 2 to 4.

In an embodiment, the number of ultraviolet emitters is 2 to 4.

In an embodiment, the number of visible light emitters is 3 to 12, preferably 7.

In an embodiment, the first surface comprises a coating, preferably an epoxy resin coating.

In an embodiment, the illumination beam of the infra-red emitter has a dispersion angle from 10° to 30°, preferably from 12 to 25°, more preferably 20°.

In an embodiment, the illumination beam of the ultraviolet emitter has a dispersion angle from 10° to 30°, preferably from 12 to 25°, more preferably from 15 to 20°.

In an embodiment, the illumination beam of the visible light emitter has a dispersion angle from 10° to 70°, preferably from 12° to 65°, more preferably from 15° to 30°; more preferably 17° to 20°.

In an embodiment, the dispersion angle illumination beam is the same on each infra-red emitter.

In an embodiment, the dispersion angle illumination beam is different on each infra-red emitter.

In an embodiment, the dispersion angle illumination beam is the same or different on each visible emitter.

In an embodiment, the plurality of light receivers is a plurality of photodiodes.

In an embodiment, the device further comprising an antenna for data transmission.

In an embodiment, the antenna extends from the second structure, being the antenna a GSM antenna.

In an embodiment, the light emitters are LEDs or lasers.

In an embodiment, the distance of the first structure and the second structure is 3 to 7cm.

In an embodiment, the distance of the first structure and the second structure is 5cm.

### BRIEF DESCRIPTION OF THE DRAWINGS

The following figures provide preferred embodiments for illustrating the disclosure and should not be seen as limiting the scope of invention.
**Figure 1****:** Schematic representation of an embodiment of the geometric disposition of the LEDs on the sensor setup wherein **1** are the LEDs and **2** are light receiver, preferably the photodetectors.
**Figure 2****:** Schematic representation of an embodiment of a LED control circuit with a NPN transistor.
**Figure 3****:** Schematic representation of an embodiment of a transimpedance Amplifier (TIA).
**Figure 4****:** Schematic representation of an embodiment of the device wherein **1** is the light emitter, **2** is the light receiver and **3** is the antenna.
**Figure 5****:** Schematic representation of the flowchart of the main program for data acquisition and treatment.
**Figure 6****:** Illustration of results of the light attenuation spectrum of different coloured dyes.
**Figure 7****:** Illustration of results of the LED attenuation graph off all water colours being Fig. 7A the results of the red dye, Fig. 7B the results for green dye, Fig. 7C the results of yellow dye and Fig. 7D is the results of blue dye.
**Figure 8****:** Illustration of results of the spectral attenuation after the addition of 33 mg/L of red dye followed by turbidity variation, using organic matter.
**Figure 9****:** Schematic representation of an embodiment where the LED emits the light beam, being represented the illuminated surface.

### DETAILED DESCRIPTION

In an embodiment, a device to evaluate the spectral transmittance of the water was developed.

In an embodiment, the device uses eleven discrete wavelengths, distributed from 360 nm to 940 nm. In an embodiment, the device comprises eleven light emitting diodes (LEDs) with similar intensities and packages (5 mm) were used, along with three photodetectors as described in Table 1.

**Table 1 - LED and Corresponding Photodiodes used in the Proposed Sensor.**

| **FOTODETETOR** | **LED** | **WAVELENGHT (nm)** | **VISUALIZATION ANGLE (º)** |
|---|---|---|---|
| GUVV-T10GD-L | MTE3661N1-UV | 360 | 20 |
| | 593-VAOL5EUV8T4 | 385 | 15 |
| BPW20RF | 749-5BWC | 430 | 20 |
| | 593-VAOL-5GSBY4 | 470 | 60 |
| | LTL2V3TGX3KS | 535 | 30 |
| | 828-OVLFY3C7 | 589 | 25 |
| | LTL353QRKNN | 639 | 12 |
| | SSL-LX5093SRC/F | 660 | 30 |
| | SSL-LX5093HD-125 | 700 | 30 |
| LTR-323DB | 720-SFH4554 | 850 | 20 |
| | 720-SFH4544 | 940 | 20 |

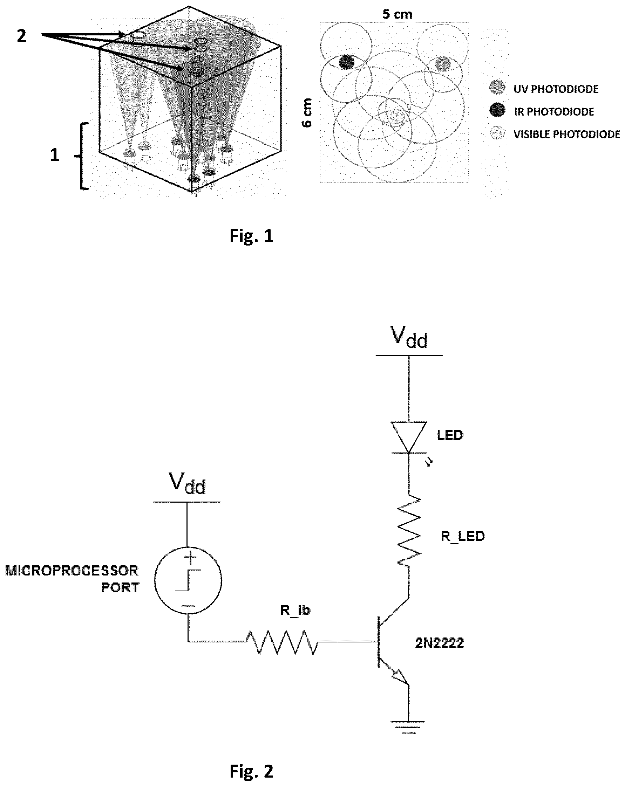

The selected LEDs have a small emission angle, resulting in a reduced illuminated surface area. While this is not an issue for the UV and IR photodetectors, which each only have two LEDs incident upon them, the visible photodetector has seven different LEDs incident upon it, and preferably, the beams should hit the surface of the photodiode. To ensure that the photodetector could cover all wavelengths, the surface areas of each LED were calculated for overlap, as depicted in **Figure 1****.**

In an embodiment, the illuminated surface area of each LED is determined by the distance between the LED and the photodetector, as well as the emission angle. As the distance between the emitter and receiver increases, there is a corresponding increase in both the loss of luminous intensity and the illuminated area. To maintain consistency, the photodiode and LED are at a distance from 3 to 7 cm, more preferably 5 cm was established between the photodiode and LED.

In an embodiment, to regulate the performance of the LEDs, a circuit with a bipolar transistor and a series resistor was implemented to control the current supplied by the microcontroller to each of the 11 LEDs. The driver circuit employed is depicted in **Figure 2****.** For the UV and visible LEDs, the operating current was approximately 30 mA. In contrast, the IR LEDs required a higher operating current of 100 mA. Considering the voltage drop, maximum current and efficiency of each LED, each RLED was calculated to have similar output in the photodiode. Adjustments of each resistor value were also performed after implementation, and similar sensibility was obtained in each color channel.

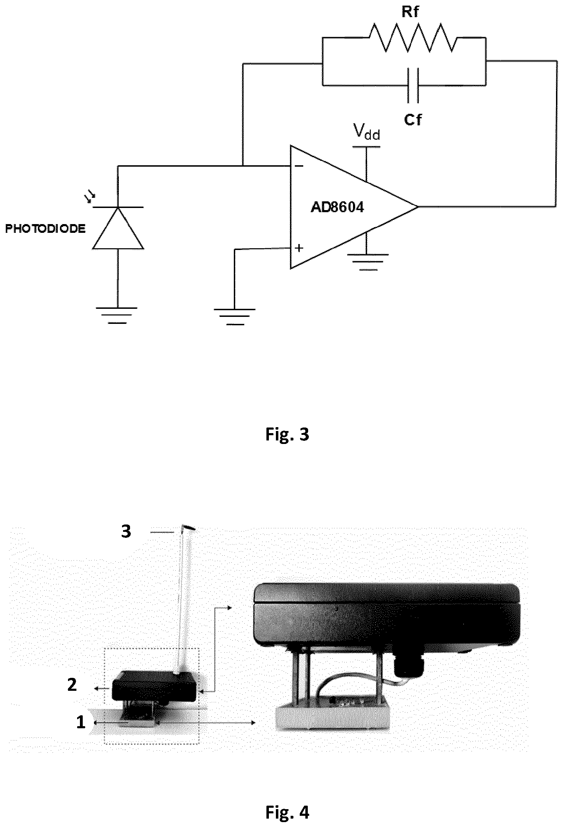

The reading circuitry was composed of a transimpedance amplifier (TIA) circuit that converted the output current of each photodetector into a corresponding voltage value, which could be measured by the analog-to-digital converter (ADC). The circuit diagram for this setup is illustrated in **Figure 3****.**

The reading circuit comprised a transimpedance amplifier (TIA) circuit that converts the output current of the photodetector into a voltage value to be read by the analog-to-digital converter (ADC). The output voltage value is determined by the opamp supply voltage. In this case, the voltage supply ranged from 0 to 5 V. The feedback resistor (Rf) determines the circuit gain, while the capacitor is utilized to create a lowpass filter that stabilizes the TIA limiting bandwidth.

To enable submersion of the sensor, the entire system was housed in an aluminum box (907-5286 RS Components, United Kingdom) with an integrated GSM antenna (Global System for Mobile Communications antenna) providing access to the main board (photodiodes, microcontroller, and GSM modem) and batteries. Preferably the antenna is Antenna GSM Quad Band 850 / 900 / 1800 / 1900 MHz 2dbi Connector SMA. Preferably, the device comprises a glass window in the box, allowing the light to reach the photodiodes. The LEDs are physically separated from the main board, enabling water to flow between them.

In an embodiment the LEDs board is insulated with a resin in manner to isolate the LEDs from water. The resin is an epoxy resin, as seen in **Figure 4****.** LEDs lens are in direct contact with water. **Figure 4** shows the location of the plurality of light emitters **1,** the location of the plurality of light receivers **2** and the antenna **3.** Preferably the plurality of light emitters **1** are LEDs or Lasers and the plurality of light receivers **2** is photodiodes.

Individual LED control was achieved by using a demultiplexer (CD74HC4514M), eliminating the need for multiple wiring between the main board and the LEDs board. To optimize space, all electronic circuits were implemented on a printed circuit board (PCB) and controlled by a microcontroller.

In an embodiment, the selected communication method was GSM (Global System for Mobile Communication). This technique allows for real-time data analysis from anywhere in the world with GSM network coverage. The user can be alerted immediately when the sensor detects any anomalies in the water color, that may indicate pollution and inform the user to take prompt action. However, the GSM module has a very high energy consumption, which requires efficient management of the sensor's autonomy.

In an embodiment, the pollution is detected when receiver detects a modification in the beams of the emitters.

In an embodiment, the implemented GSM module was the SIM900A, which contains a socket for a SIM card and an antenna (powered with 5 V and with consumption peaks of 1 A). The module is only turned on when communication is needed to minimize energy consumption. It is controlled through a serial port from the microcontroller. The information is then stored on the free-to-use ThingSpeak online platform, which also allows for data export in Excel format.

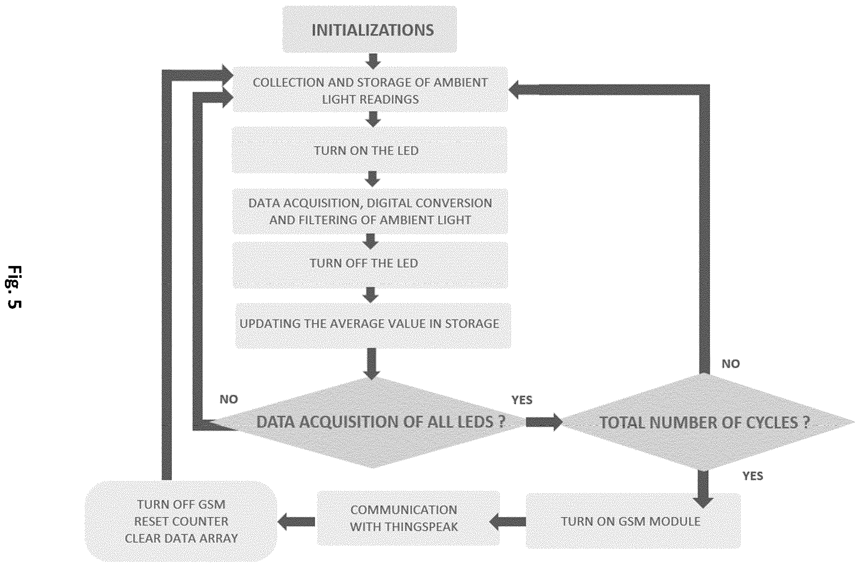

The acquired values are stored in a vector with twelve positions. Each time a new reading is taken, the new value is added to the existing value and an arithmetic mean is calculated. Preferably it is calculated the value average. This results in a vector containing dynamic average values for each reading. Subsequently, the GSM module is activated, and the values within the vector are communicated to the server. Finally, the GSM module is deactivated, the data vector is cleared, and the auxiliary counter is reset. These steps are represented in the form of a flowchart, as depicted in the following **Figure 5****.** The method comprises the following steps:
a) collecting and storing ambient light readings by the light receiver, preferably by a photodetector;
b) Turning on one light emitter, preferably a LED, and sequentially turning on the following light emitters;
c) Data acquisition, digital conversion and filtering of ambient light;
d) Turning off the light emitters;
e) Updating the average value in storage.

If the data acquisition of all the LEDs was done and the total number of cycles was reached, then tur on the GSM module, communicate with thingspeak and turn off the GSM, reset counter and clear data array.

If the data acquisition of all the LEDs was not done, then the steps a) to e) are repeated.

The tests were carried out with the sensor submerged, and the antenna uncovered to enable data transmission.

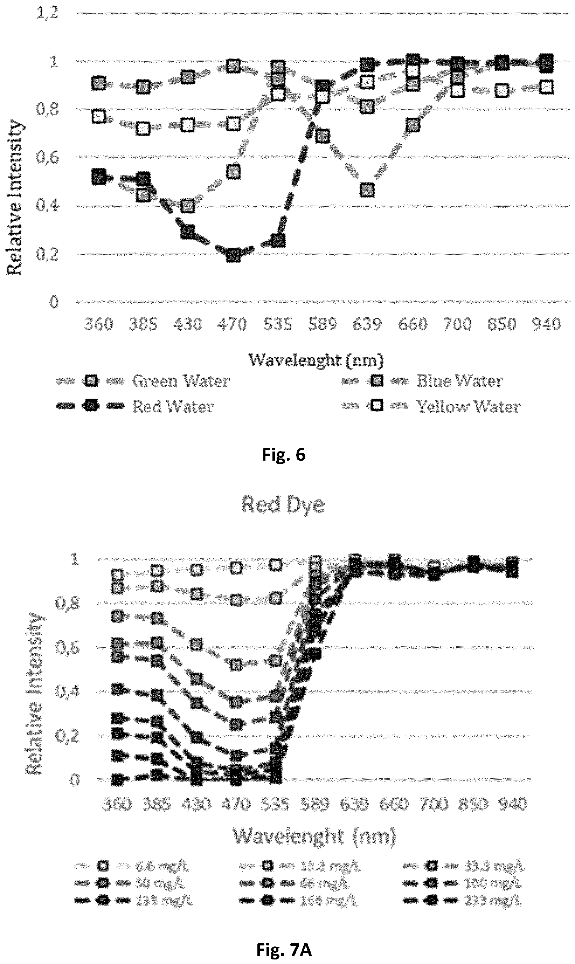

For the colorimetry tests, initially, clean water was used to create the reference baseline (for 2 hours), followed by using four different colored dyes (blue, green, red, and yellow) 1.5 ml to analyze absorption (for another 2 hours). 300 readings were analyzed for each LED. The data from the different LEDs were compiled in **Figure 6****.** In vertical axis is plotted the percentage of transmitted light, using as reference the light transmitted in clear water.

Water with different color dyes have different spectral transmission, as expected. Red dye increases absorption in UV to green wavelength; blue dye increases absorption in yellow to red wavelengths a green dye increases absorption in UV to blue light. This is obtained with the used dies, but different results can be obtained with different dyes. The yellow die has lower light attenuation, mainly from UV to Orange wavelengths.

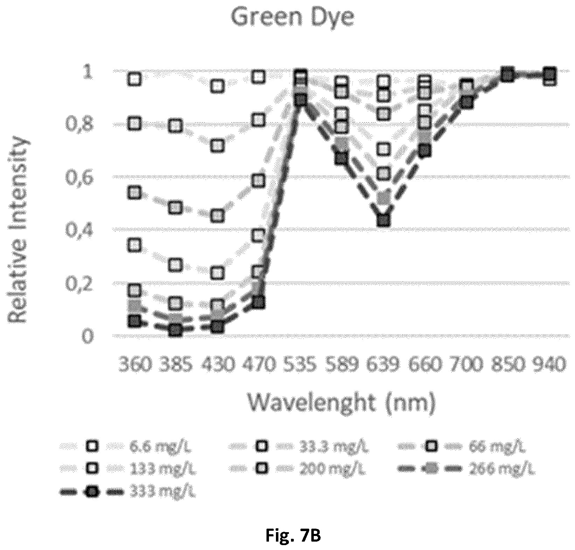

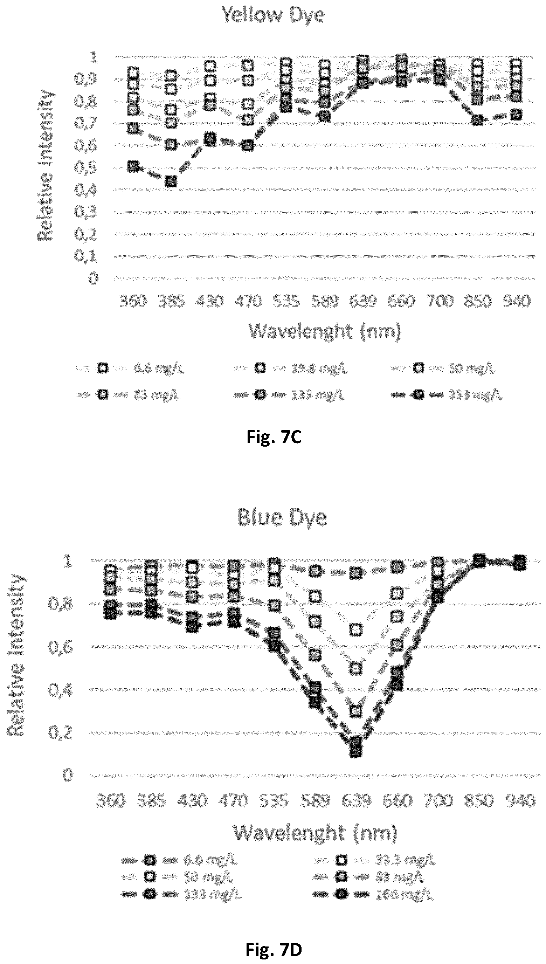

Next, in an embodiment, the concentration of each dye was varied between 6.6 mg/L (100 mg dye in 15 L of water) to 333 mg/L, for each of the available dyes. Upon analysing these tests, clear attenuation patterns can be observed across different colours. **Figure 7** presents the light transmission (calculated as a percentage to light transmitted in clear water), when dye was added, for each dye color.

Attenuation increases with dye concentration in all cases. In the case of yellow, there is a nearly uniform attenuation across all wavelengths, with a greater emphasis on shorter wavelengths. Notably, its distinct advantage over other colors lies in its ability to attenuate infrared light effectively. The red color exhibits a more pronounced attenuation of lower wavelengths while exhibiting minimal attenuation at higher wavelengths. Conversely, the blue color demonstrates significant attenuation of orange wavelengths. The green color, on the other hand, appears to be a composite of attenuation signals from both red and blue, albeit without attenuation in the green LED. Based on this comprehensive information, it can be concluded that all four of these colors can be detected by this sensor, given their well-defined attenuation patterns. However, detecting lower concentrations, typically below 6.6 mg/L, poses greater challenges, due to lower sensibility, which can be solved with more sensible light receivers.

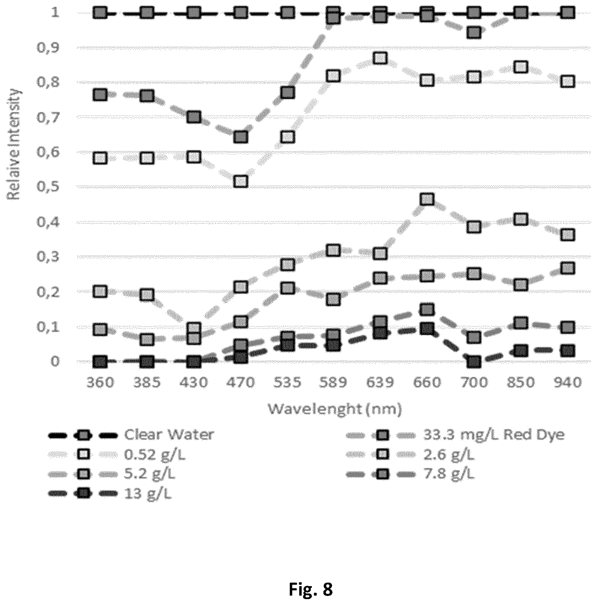

The ability to detect color changes was also demonstrated with water of high turbidity, to emulate river condition during rainy days, real soil was employed and dissolved in varying concentrations within a specified volume of water. **Figure 8** represents the transmission measured in the water, with different quantities of added soil.

The attenuation increases as the concentration (turbidity) of soil rises. An increased attenuation is particularly noticeable for UV and IR wavelengths. Furthermore, tests were conducted to assess turbidity variation in dye-infused water. **Figure 8** shows the transmission of water containing a concentration of 33.3 mg/L of red dye, followed by variations in the concentration of organic matter ranging from 0.52 g/L to 7.8 g/L

At low turbidity levels (up to 5.2 g/L), the colour pattern remains clearly visible. However, as turbidity increases above, the pattern becomes less pronounced, but the different attenuation in several wavelengths is still visible, despite the higher attenuation at the last 3 wavelengths (700, 850 nm and 940 nm).

An analysis was carried out on the effect of ambient light, and it was observed that direct exposure to sunlight saturates the detection system, thus the sensor should be placed in areas with minimal solar exposure. A transmission methodology covering the spectrum of 365-940 nm was utilized, which enables real-time communication via GSM, providing the user with analysis data. Energy consumption can be significantly reduced by using a more efficient microcontroller (STM32 Ultra low power), allowing for longer reading periods. Turbidity and coloration tests with dye have shown that even at higher turbidity levels (such as during rainfall and soil erosion), the sensor is capable of reading colour differences.

To enhance detection capabilities at lower concentrations, the resolution of the analog-to-digital converter (ADC) can be increased and more more sensitive light receivers, preferably photodiodes, can be used.

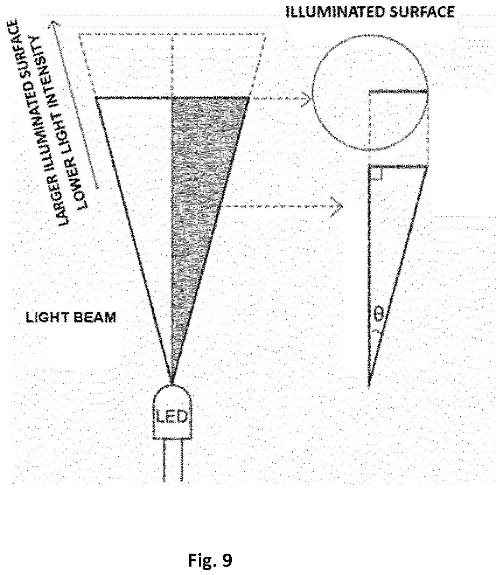

**Figure 9** shows an embodiment where the LED emits the light beam, being represented the illuminated surface. Where is larger illuminated surface there is a lower light intensity.

The term "comprising" whenever used in this document is intended to indicate the presence of stated features, integers, steps, components, but not to preclude the presence or addition of one or more other features, integers, steps, components or groups thereof.

The disclosure should not be seen in any way restricted to the embodiments described and a person with ordinary skill in the art will foresee many possibilities to modifications thereof. The above described embodiments are combinable.

The following claims further set out particular embodiments of the disclosure.

### References

[1] D. Tickner, H. Parker, C. R. Moncrieff, N. E. M. Oates, E. Ludi, and M. Acreman, "Managing Rivers for Multiple Benefits-A Coherent Approach to Research, Policy and Planning," Front. Environ. Sci., vol. 5, no. FEB, p. 4, Feb. 2017, doi: 10.3389/fenvs.2017.00004.
[2] Z. Xu, J. Xu, H. Yin, W. Jin, H. Li, and Z. He, "Urban river pollution control in developing countries," Nature Sustainability, vol. 2, no. 3. Nature Publishing Group, pp. 158-160, Mar. 01, 2019, doi: 10.1038/s41893-019-0249-7.
[3] "European waters -- Assessment of status and pressures 2018 - European Environment Agency." https://www.eea.europa.eu/publications/state-of-water (accessed May 23, 2023).
[4] C. Li, J. C. Gui, L. Y. Sun, W. X. Cao, Z. H. Sun, and Y. Z. Yang, "In-situ instrument for rapid detection of nitrates and nitrites in water," Guang Pu Xue Yu Guang Pu Fen Xi/Spectroscopy Spectr. Anal., vol. 33, no. 6, pp. 1714-1718, Jun. 2013, doi: 10.3964/j.issn.1000-0593(2013)06-1714-05.
[5] E. T. Steimle, E. A. Kaltenbacher, and R. H. Byrne, "In situ nitrite measurements using a compact spectrophotometric analysis system," Mar. Chem., vol. 77, no. 4, pp. 255-262, Mar. 2002, doi: 10.1016/50304-4203(02)00003-8.
[6] B. Lin, J. Xu, K. Lin, M. Li, and M. Lu, "Low-Cost Automatic Sensor for in Situ Colorimetric Detection of Phosphate and Nitrite in Agricultural Water," ACS Sensors, vol. 3, no. 12, pp. 2541-2549, Dec. 2018, doi: 10.1021/acssensors.8b00781.
[7] Y. Xing et al., "A cellphone-based colorimetric multi-channel sensor for water environmental monitoring," Front. Environ. Sci. Eng., vol. 16, no. 12, pp. 1-12, Dec. 2022, doi: 10.1007/s11783-022-1590-z.
[8] V. E. Brando and A. G. Dekker, "Satellite hyperspectral remote sensing for estimating estuarine and coastal water quality," IEEE Trans. Geosci. Remote Sens., vol. 41, no. 6 PART I, pp. 1378-1387, Jun. 2003, doi: 10.1109/TGRS.2003.812907.
[9] G. C. Anzalone, A. G. Glover, and J. M. Pearce, "Open-Source Colorimeter," Sensors, vol. 13, no. 4, pp. 5338-5346, Apr. 2013, doi: 10.3390/s130405338.
[10] K. Devarayan, M. Kathavarayan, A. Theivasigamani, M. Sukumaran, and S. Kandasamy, "Development of portable colorimeter for on-site determination of water quality in aquaculture," Pigment Resin Technol., vol. ahead-of-print, no. ahead-of-print, 2022, doi: 10.1108/PRT-12-2021-0140.
[11] J. Asheim, E. V. Kvittingen, L. Kvittingen, and R. Verley, "A simple, small-scale lego colorimeter with a light-emitting diode (LED) used as detector," J. Chem. Educ., vol. 91, no. 7, pp. 1037-1039, Jul. 2014, doi: 10.1021/ed400838n.

## Claims

1. Optical device for measuring turbidity and colour content of fresh water or sea water comprising
a first structure comprising on its top surface a plurality of light emitters;
a second structure comprising a plurality of light receivers;
wherein said plurality of light emitters and plurality of light receivers comprise:
at least one infra-red emitter and one infra-red receiver;
at least one ultraviolet emitter and one ultraviolet receiver;
at least one visible emitter and one visible receiver;
wherein each light emitter emits an illumination beam with an angle from 10 to 80°;
wherein the first structure and the second structure are at a pre-determined distance;
wherein the first structure and the second structure form a chamber for receiving the fluvial or sea water.

2. Optical device according to the previous claim wherein the number of infra-red emitter is 2 to 4.

3. Optical device according to any of the previous claims wherein the number of ultraviolet emitters is 2 to 4.

4. Optical device according to any of the previous claims wherein the number of visible light emitters is 3 to 12, preferably 7.

5. Optical device according to any of the previous claims wherein the first surface comprises a coating, preferably an epoxy resin coating.

6. Optical device according to any of the previous claims wherein the illumination beam of the infra-red emitter has a dispersion angle from 10° to 30°, preferably from 12 to 25°, more preferably 20°.

7. Optical device according to any of the previous claims wherein the illumination beam of the ultraviolet emitter has a dispersion angle from 10° to 30°, preferably from 12 to 25°, more preferably from 15 to 20°.

8. Optical device according to any of the previous claims wherein the illumination beam of the visible light emitter has a dispersion angle from 10° to 70°, preferably from 12° to 65°, more preferably from 15° to 30°; more preferably 17° to 20°.

9. Optical device according to any of the previous claims wherein the dispersion angle of the illumination beam is the same on each infra-red emitter.

10. Optical device according to any of the previous claims 1-8 wherein the angle illumination beam is different on each infra-red emitter.

11. Optical device according to any of the previous claims wherein the angle illumination beam is the same or different on each visible emitter.

12. Optical device according to any of the previous claims wherein the plurality of light receivers is a plurality of photodiodes.

13. Optical device according to any of the previous claims further comprising an antenna for data transmission extending from the second structure.

14. Optical device according to any of the previous claims wherein the first structure and the second structure are at a distance of 3 to 7 cm, preferably 5 cm.

15. Optical device according to any of the previous claims wherein the light emitters are LEDs or lasers.
